# EUROPÄISCHE PATENTSCHRIFT

(11) **EP 1 023 008 B1**
(45) Veröffentlichungstag und Bekanntmachung des Hinweises auf die Patenterteilung: **10.12.2003**
(21) Anmeldenummer: 98955514.9
(22) Anmeldetag: 22.10.1998
(51) Int. Cl.: A61F 2/06

(54) **AUFGEWEITETER STENT UND VERFAHREN ZUM HERSTELLEN DESSELBEN**
EXPANDED SPREADER AND A METHOD FOR PRODUCING THE SAME
EXTENSEUR ELARGI ET SON PROCEDE DE FABRICATION

(30) Priorität: 23.10.1997 DE 19746882
(43) Veröffentlichungstag der Anmeldung: 02.08.2000
(73) Patentinhaber: Angiomed GmbH & Co. Medizintechnik KG, 76227 Karlsruhe (DE)
(72) Erfinder: ZSCHEEG, Harry, D-69121 Heidelberg (DE)
(74) Vertreter: HOFFMANN - EITLE
(86) Internationale Anmeldenummer: EP9806717
(87) Internationale Veröffentlichungsnummer: WO99021509

(56) Entgegenhaltungen:
- EP-A- 0 688 545
- EP-A- 0 712 614
- DE-A- 4 418 336
- US-A- 5 514 154

## Beschreibung

### TECHNISCHES GEBIET

Die vorliegende Erfindung betrifft expandierbare Stents zum Einsetzen in röhrenförmige anatomische Strukturen, wie Gallengang, Leber, Arterien, Speiseröhre, Luftröhre o.ä., sowie ein Verfahren zum Herstellen eines solchen Stents. Im folgenden werden solche röhrenförmigen anatomischen Strukturen beispielhaft mit Gefäße oder Körpergefäß bezeichnet.

### STAND DER TECHNIK

Gattungsgemäße Stents können aus Kunststoff oder aus inertem Metall wie z.B. Stahl oder Metallegierungen bestehen. Typische Einsatzgebiete sind die Erweiterung eines Harnleiters im Prostatabereich bei benigner Prostata-Hyperplasie (BPH) oder auch in pathologisch verengten Blutgefäßen zur Erweiterung und Offenhaltung dieser Gefäße. Sie weisen eine Gitterstruktur auf, die aus Wandsegmenten und zwischen diesen Wandsegmenten gebildeten Öffnungen besteht. Diese Struktur ermöglicht, daß der Stent durch das Gewebe des Gefäßes, in das er eingesetzt ist, umwachsen werden kann. Es ist bekannt, Stents in Form einer spiralförmig gewundenen Wendel auszubilden; sie können aus gewebtem oder gestricktem Draht- oder Kunststoffmaterial bestehen. Gattungsgemäße Stents können zum einen dauerhaft plastisch verformbar (ballonexpandierbare Stents), zum anderen elastisch oder superelastisch sein (selbstexpandierende Stents)oder Formgedächtnis- oder shape memory-Eigenschaften aufweisen (ebenfalls selbstexpandierende Stents), wie sie beispielsweise bei bestimmten Nickel-Titan-Legierungen (Nitinol®) gegeben sind.

Es ist häufig wünschenswert, gattungsgemäße Stents in Gefäßkrümmungen einzusetzen. Derartige Gefäßkrümmungen können sehr geringe Krümmungsradien aufweisen. Für diesen Anwendungsbereich sind hochflexible Stents entwickelt worden. So offenbart z.B. die DE 43 03 181 A1 einen implantierbaren Stent, der eine höhere Flexibilität und Biegsamkeit sowie eine bessere Stabilität bei Verbiegung aufweist als zu ihm vorbekannte Stents. Diese Eigenschaften werden dadurch erreicht, daß er mehrere in Achsrichtung hintereinander angeordnete, sich über seinen Umfang erstreckende Mäanderbahnen aufweist. Diese Mäanderbahnen sind durch Verbindungsabschnitte miteinander verbunden, wobei aber in Umfangsrichtung zwischen einander zugewandten Verbindungsabschnitten mindestens jeweils zwei nicht miteinander verbundene, einander zugewandte Verbindungsabschnitte angeordnet sind. Diese Auflösung einer festen Gitterstruktur erzielt die gewünschten Festigkeitseigenschaften. Bei Anwendung dieses bekannten Stents in sehr eng gekrümmten Gefäßbereichen kann es jedoch vorkommen, daß die Kanten der nicht miteinander verbundenen Verbindungsabschnitte derart in das innere Lumen der röhrenförmigen Struktur ragen, daß die nutzbare Querschnittsfläche des Stents verringert wird.

Es sind ferner Stents bekannt, bei denen zur Verbesserung der Verankerung des Stents in dem umgebenden Gefäßgewebe die Endbereiche des Stents verdickt sind, so daß der Stent keinen über seine ganze Länge einheitlichen Außendurchmesser aufweist. Die nach außen vorstehenden verdickten Enden werden von dem Gefäßgewebe umwachsen und verhindern eine Lageveränderung des Stents in seiner Axialrichtung. Eine solche Ausführungsform ist beispielsweise aus der EP 0 778 011 A2 bekannt.

Um eine solche Lageveränderung durch andere Mittel zu verhindern, ist in der WO 86/02211 ebenfalls vorgeschlagen worden, eine Gitterstruktur eines Stents zusätzlich mit in Richtung des Gefäßgewebes vorstehenden Widerhaken zu versehen. Die grundlegende Gitterstruktur dieses Stents wird hierdurch jedoch nicht verändert.

Ein Stent mit den Merkmalen des Oberbegriffs den Ansprüchs 1 ist beispielsweise aus der DE-A-441833 bekannt.

### DARSTELLUNG DER ERFINDUNG

Es ist Aufgabe der vorliegenden Erfindung, einen expandierbaren Stent bereitzustellen, der höchsten Anforderungen an Lagestabilität genügt und der für Anwendungsgebiete geeignet ist, in denen er starken Krümmungen ausgesetzt ist. Es ist weiterhin Aufgabe der vorliegenden Erfindung, ein Herstellungsverfahren für einen Stent bereitzustellen, der diesen Aufgaben genügt.

Diese Aufgaben werden vorteilhafterweise durch einen expandierbaren Stent mit den in Anspruch 1 definierten Merkmalen sowie durch ein Verfahren mit den in Anspruch 12 definierten Schritten gelöst. Vorteilhafte Ausführungsformen bzw. Weiterbildung des expandierbaren Stents ergeben sich aus den Unteransprüchen 2 - 11 und des erfindungsgemäßen Herstellungsverfahrens aus den Unteransprüchen 13 - 22.

So umfaßt ein erfindungsgemäß expandierbarer Stent eine elastische rohrförmige Gitterstruktur mit einem ersten Endbereich, einem zweiten Endbereich, einer Längsrichtung und einer Radialrichtung. Diese Gitterstruktur bestimmt einen Außendurchmesser und ein inneres Lumen. Sie wird von Wandsegmenten gebildet, wobei sich diese Wandsegmente an Kreuzungen verzweigen. Ferner ist die Gitterstruktur an zumindest einigen dieser Kreuzungen unterbrochen, womit die Flexibilität des Stents erhöht wird. Der erfindungsgemäße expandierbare Stent ist dadurch gekennzeichnet, daß die Wandsegmente zumindest an den unterbrochenen Kreuzungen in der Radialrichtung des Stents aufgeweitet sind. Diese Aufweitung ist folglich zu dem den Stent umgebenden Gefäßgewebe hin gerichtet, wobei die unterbrochenen Kreuzungen zwischen dem ersten Endbereich und dem zweiten Endbereich der Gitterstruktur liegen. Die Aufweitung in Radialrichtung bewirkt, daß auch bei extremer Krümmung des Stents entlang der Längsrichtung das innere Lumen des Gefäßes nicht dadurch verkleinert wird, daß Wandsegmente an den unterbrochenen Kreuzungen in das innere Lumen ragen. Die erfindungsgemäße Lösung bietet mehrere Vorteile. So ist es einerseits möglich, den erfindungsgemäßen Stent unabhängig von der Krümmung der zu behandelnden Körperstelle mittels eines Ballonkatheters zu expandieren und somit zu implantieren oder das dortige Gefäßgewebe mit bereits implantierten Stents mit Ballonkathetern zu behandeln, andererseits, den Stent als selbstexpandierenden Stent auszubilden. Eine lokale Verkleinerung des inneren Lumens könnte ferner zu unkontrollierten Strombahnwirbeln in der den Stent durchströmenden Flüssigkeit und somit zu neuen Verschlußreaktionen führen. Dies wird durch die erfindungsgemäße Lösung ebenfalls verhindert. Zugleich wird durch die Aufweitung der Wandsegmente an den unterbrochenen Kreuzungen in der Radialrichtung des Stents die Verankerung des Stents in dem umgebenden Gefäßgewebe verbessert, so daß der Stent seine Lage nicht verändern kann, nachdem er implantiert wurde. Es wird ersichtlich sein, daß je nach gewähltem Fertigungsverfahren für den Stent auch die Wandsegmente an den nicht unterbrochenen Kreuzungen aufgeweitet sein können.

Gemäß einer vorteilhaften Weiterbildung sind weiterhin die Wandsegmente in dem ersten und/oder dem zweiten Endbereich in der Radialrichtung aufgeweitet, wodurch die Verankerung unterstützt und Abschlüsse des Stents erzielt werden, die nach außen gerichtet sind. Konische beziehungsweise bauchige Formen des Stents sind folglich möglich. Gleichmäßige Strömungsübergänge ohne Totwassergebiete sind sichergestellt. Um die Aufweitung der Wandsegmente ebenfalls gleichmäßig und bezüglich der Strömungsverhältnisse optimal zu gestalten, ist sie durch eine bogenförmige Krümmung dieser Wandsegmente entlang der Längsrichtung gebildet. Im Umfang der vorliegenden Erfindung ist ebenfalls vorgesehen, die Aufweitungen z.B. durch Abwinkeln zu erzeugen, was allerdings im Vergleich zu den bogenförmigen Krümmungen weniger bevorzugt ist.

Vorteilhafterweise ist der Stent derart weitergebildet, daß die Wandsegmente in einer regelmäßigen Verteilung über den Stent im wesentlichen an 2/3 aller Kreuzungen unterbrochen sind. Diese Verteilung ist regelmäßig sowohl in Umfangsrichtung als auch in Axialrichtung des Stents und das genaue Muster der Verteilung hängt von der gewählten Form der Gitterstruktur ab. Es hat sich herausgestellt, daß eine Unterbrechung von im wesentlichen 2/3 aller Kreuzungen einen guten Kompromiß darstellt zwischen einer hohen Festigkeit des Stents einerseits und guter Flexibilität und Stabilität bei Verformung des Stents andererseits. Die somit erzielte Flexibilität kann je nach medizinischer Indikation auch nach Wunsch verändert werden, indem eine entsprechend unterschiedliche Anzahl von Kreuzungen unterbrochen ist.

Wie bereits erwähnt, weist die Gitterstruktur Öffnungen auf. Um bei kollateralen Aterienabzweigungen das Sicherstellen einer regionalen Weiterversorgung der Gefäße zu verbessern, bis eine Intimaneubildung erfolgt, weisen diese Öffnungen im expandierten Zustand des Stents vorteilhafterweise eine Öffnungsweite von maximal 9 mm auf.

Um die Reokklusionsgefahr des Gefäßgewebes an der Implantatstelle so gering wie möglich zu halten, muß der Stent eine gewisse Aufstellkraft aufweisen. Für diese Aufstellkraft ist es besonders günstig, wenn die Wandsegmente eine Breite zwischen 0,12 mm und 0,17 mm aufweisen. Unter Breite wird die geringste Erstreckung der Wandsegmente von der dem Wandsegment auf einer Seite benachbarten Öffnung der Gitterstruktur zur auf der gegenüberliegenden Seite benachbarten Öffnung der Gitterstruktur, im wesentlichen in Umfangsrichtung des Stents verstanden.

Um die günstigen Eigenschaften eines no-profile oder eines low-profile Stents vorteilhaft weiterzubilden, weist die Gitterstruktur in der Radialrichtung im wesentlichen eine Wandstärke von zwischen 0,2 mm und 0,3 mm auf. Da diese Wandstärke im Verlauf der Längsrichtung des Stents je nach Herstellungsverfahren geringfügig schwanken kann, wird die vorstehende Angabe als eine charakteristische Wandstärke verstanden. Gemäß einer weiteren vorteilhaften Ausgestaltung des erfindungsgemäßen Stents besteht dieser aus einem metallischen Werkstoff mit Formgedächtnis. Dieser Werkstoff mit Formgedächtnis- oder shape memory-Effekt besteht weiterhin vorteilhafterweise aus einer Nickel-Titan-Legierung. Die Legierungsanteile, die insbesondere vorteilhaft gemäß den Angaben in Anspruch 11 enthalten sind, ermöglichen es, den Stent auf Temperatur reagierend zu gestalten, so daß dieser im nicht expandierten Zustand in den Körper eingeführt werden kann, sich unter Körpertemperatur beim Plazieren auf das gewünschte Maß ausdehnt und sich an der gewünschten zu behandelnden Stelle eigenständig implantiert.

Das erfindungsgemäße Herstellungsverfahren für einen Stent sieht vor, daß ein rohrförmiges Element mit einem Außendurchmesser, einem inneren Lumen, einem ersten Endbereich und einem zweiten Endbereich bereitgestellt wird. Weiterhin wird das rohrförmige Element zu einer Gitterstruktur geschlitzt. Unter Schlitzen soll Erzeugen von schlitzähnlichen Öffnungen durch mechanische -z.B. Stanzen-, elektromagnetische oder elektrische -z.B. Laserschneiden oder Funkenerosion- oder chemische Fertigungsverfahren -z.B. Ätzen- verstanden werden. Diese sich aus dem Schlitzen ergebende Gitterstruktur wird aus Wandsegmenten gebildet, die sich an Kreuzungen verzweigen. In einem weiteren Schritt werden zumindest einige der Kreuzungen an ausgewählten Positionen unterbrochen, wodurch die Flexibilität des Stents erhöht wird. Je nach gewähltem Fertigungsverfahren ist es u.U. vorteilhaft, die Schritte des Schlitzens und des Unterbrechens gleichzeitig durchzuführen. Zusätzlich werden die Wandsegmente an den unterbrochenen Kreuzungen in der Radialrichtung derart aufgeweitet, daß bei Krümmung des Stents entlang der Längsrichtung eine Verkleinerung des inneren Lumens durch die Wandsegmente an den unterbrochenen Kreuzungen verhindert ist. Auch hier ist ersichtlich, daß es vorteilhaft sein kann, auch die Wandsegmente an den nicht unterbrochenen Kreuzungen aufzuweiten.

Erfindungsgemäß ist weiterhin vorteilhafterweise vorgesehen, daß der Schritt des Aufweitens ebenfalls das Aufweiten der Wandsegmente in der Radialrichtung an dem ersten und dem zweiten Endbereich beinhaltet.

Es hat sich als besonders verfahrensökonomisch herausgestellt, die erfindungsgemäßen Verfahrensschritte in der in Anspruch 12 angeführten Reihenfolge durchzuführen.

Gemäß einer vorteilhaften Weiterbildung des erfindungsgemäßen Verfahrens ist weiterhin vorgesehen, daß nach dem Schlitzen des rohrförmigen Elementes und vor dem Unterbrechen der Kreuzungen ein Schritt enthalten ist, in dem die Struktur des Metallgitters des Stents beeinflußt wird, um den Formgedächtniseffekt zu programmieren.

Um die Strömungsverhältnisse in und um den Stent günstig zu beeinflussen, ist weiterhin erfindungsgemäß vorgesehen, daß das Herstellungsverfahren durch einen Schritt abgeschlossen wird, in dem der Stent poliert wird. Durch das Polieren wird die Oberfläche extrem glatt und eventuell vorhandene oder entstandene Kratzer, Kanten oder allgemein Oberflächenunregelmäßigkeiten werden beseitigt.

Vorteilhaft ist erfindungsgemäß ebenfalls vorgesehen, daß der Schritt des Aufweitens mehrere Teilschritte umfaßt, die idealerweise in der angegebenen Reihenfolge vorgenommen werden. Demzufolge wird zum einen der Stent auf einen Dorn aufgebracht, wobei der Dorn als Gegenform zur aufgeweiteten Form des Stents ausgebildet ist. Dieses Positiv-Negativ-Verhältnis der Formen dient dazu, dem Stent die Form des Dorns zu geben. Die Abmessungen des Dorns entsprechen dabei im wesentlichen den Abmessungen des Stents im expandierten Zustand. Nachdem der Stent auf den Dorn gebracht wurde, wird der Stent, oder auch der Stent und der Dorn, erhitzt. Nachfolgend wird der Stent und ggfs. der Dorn wieder abgekühlt und der abgekühlte Stent wird von dem Dorn entfernt. Die Aufheiz- und Abkühlschritte können vorteilhafterweise durch einen aufheizbaren sowie kühlbaren Dorn vorgenommen werden. Andererseits ist es denkbar, den Stent von außen zu erhitzen. Es wird gewährleistet, daß der Stent als ganzes, mit den aufgeweiteten Wandsegmenten, ein temperaturreaktives Verhalten zeigt.

Es ist gleichfalls möglich, die gewünschte Form des Stents von außen zu erzeugen, indem beispielsweise in der Radialrichtung außerhalb des Stents ein äußeres Formelement als Gegenform vorgesehen ist, das in seiner Kontur der aufgeweiteten Form des Stents entspricht. Besonders wünschenswert ist es, den Stent derart zu formen, daß ein Dorn in Kombination mit einem derartigen Formelement benutzt wird.

In bereits geschilderter Art besteht der Stent vorteilhafterweise aus einem metallischen Werkstoff. Gemäß einer bevorzugten Ausführungsform des erfindungsgemäßen Verfahrens weist dieser Werkstoff eine Schwellentemperatur auf, bei der sich Versetzungen im Werkstoff des Stents neu orientieren können. Der Stent wird erfindungsgemäß in dem Teilschritt des Erhitzens auf eine Temperatur oberhalb dieser Versetzungsschwellenentemperatur erhitzt und in dem Schritt des Abkühlens auf eine Temperatur unterhalb dieser Versetzungsschwellentemperatur abgekühlt. Hierdurch ist ermöglicht, die temperaturreaktiven Eigenschaften des Stents exakt auf die Gegebenheiten bezüglich z.B. der menschlichen Körpertemperatur einzustellen. Der Formgedächtniseffekt beziehungsweise das Formerinnerungsvermögen wird folglich mit seinen Reaktionsparametern programmiert.

### KURZE BESCHREIBUNG DER ZEICHNUNGEN

Im folgenden werden zur weiteren Erläuterung und zum besseren Verständnis der Erfindung unter Bezugnahme auf die beigefügten Zeichnungen Ausführungsbeispiele der Erfindung näher beschrieben und ausgeführt. Es zeigt:
- Fig. 1: einen Stent gemäß dem Stand der Technik, wobei in der Figur charakteristische Größen von gattungsgemäßen Stents zur Anwendung bei einer bestimmten medizinischen Indikation gekennzeichnet sind;
- Fig. 2: eine Einzelheit eines konventionellen Stents gemäß der Fig. 1, die dort mit II gekennzeichnet ist;
- Fig. 3: eine Seitenansicht eines erfindungsgemäßen Stents gemäß einer ersten Ausführungsform;
- Fig. 4: einen vergrößerten Ausschnitt aus der Fig. 3;
- Fig. 5: eine Seitenansicht eines erfindungsgemäßen Stents nach einer zweiten Ausführungsform;
- Fig. 6: eine Einrichtung, die gemäß einer bevorzugten Ausführungsform beim erfindungsgemäßen Verfahren zum Einsatz kommt; und
- Fig. 7: schematisch einen erfindungsgemäßen Stent in eingesetztem Zustand, wobei der Stent an der Stelle einer starken Krümmung eingesetzt ist.

### BESCHREIBUNG VON AUSFÜHRUNGSBEISPIELEN

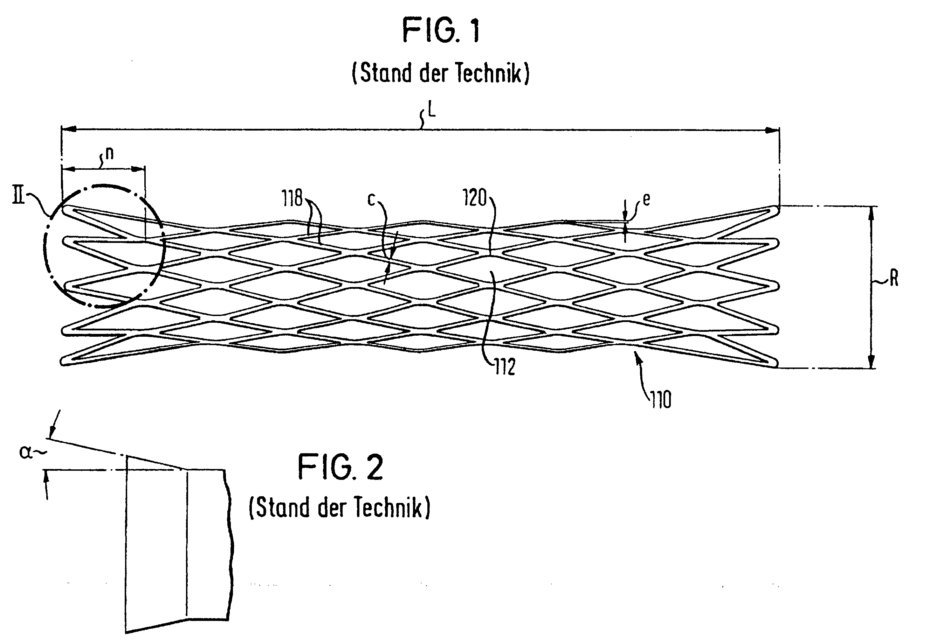

Fig. 1 zeigt eine Seitenansicht eines konventionellen gattungsgemäßen Stents. Aus der Seitenansicht sind übliche Größenverhältnisse des Stents zur Anwendung in der Karotis entnehmbar. Unter anderem weisen solche Stents eine Längsrichtung L und eine Radialrichtung R auf und bestimmen ferner ein inneres Lumen.

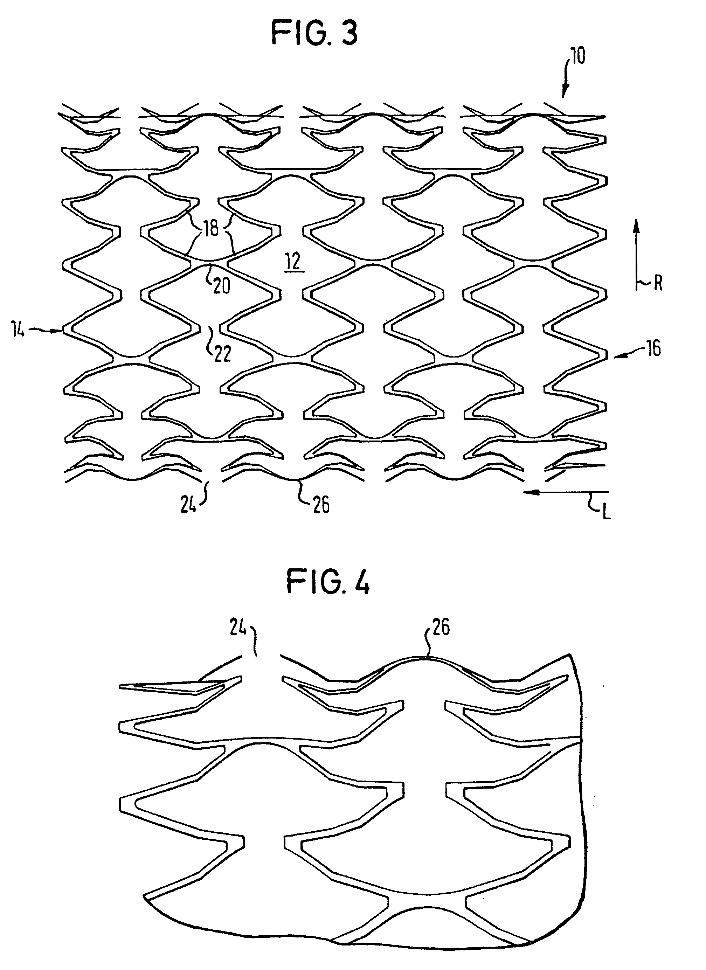

Für den erfindungsgemäßen Stent, der in den Fig. 3 bis 5 dargestellt ist, sind für die medizinische Indikation des Einsatzes in die Karotis (Halsschlagader) bestimmte Abmessungen besonders vorteilhaft. Zur Einfachheit der Darstellung sind die Einzelheiten, deren Abmessungen für den erfindungsgemäßen Stent wesentlich sind, an korrespondierenden Einzelheiten des in der Fig. 1 dargestellten Stents gemäß dem Stand der Technik gekennzeichnet.

Der Fig. 1 ist entnehmbar, daß der konventionelle Stent 110 aus einer Gitterstruktur 112 besteht. Diese Gitterstruktur wird durch verschiedene Wandsegmente 118 gebildet, die sich an Kreuzungen 120 verzweigen. Die Länge der Wandsegmente von Kreuzung zu Kreuzung ist mit n und die Breite in Umfangsrichtung mit c bezeichnet. Die Wanddicke in Radialrichtung ist mit e gekennzeichnet.

Aus der Fig. 2 ist entnehmbar, unter welchem Winkel α die Wandsegemte 118 an den Kreuzungen 120 von der Längsrichtung L des Stents abweichen. Die Länge n der Wandsegmente von Kreuzung zu Kreuzung in Kombination mit dem Winkel α bestimmt die maximale Öffnungsweite, die bei dem dargestellten Ausführungsbeispiel in Umfangsrichtung zwischen den Kreuzungen gebildet wird.

Fig. 3 zeigt ein erstes Ausführungsbeispiel eines erfindungsgemäßen Stents 10. Dieser erfindungsgemäße Stent weist eine Radialrichtung R und eine Längsrichtung L und ferner einen ersten Endbereich 14 und einen zweiten Endbereich 16 auf. Die charakteristischen Radial- bzw. Längsabmessungen des Stents 10 unterscheiden sich von denen des in der Fig. 1 dargestellten konventionellen Stents 110. Diese Abmessungen werden je nach medizinischer Indikation gewählt. Der erfindungsgemäße Stent 10 (Fig. 3) ist durch eine Gitterstruktur 12 gebildet. Diese Gitterstruktur weist mehrere Wandsegmente 18 auf, die sich an Kreuzungen verzweigen. Von diesen Kreuzungen sind einige 22 unterbrochen, andere 20 nicht. Das Unterbrechen einiger Kreuzungen 22 in einer regelmäßigen Verteilung über den Stent 10 ermöglicht es, die Flexibilität des gesamten Stents zu erhöhen. Je nach medizinischer Indikation sind entsprechend viele Kreuzungen in regelmäßiger Verteilung über den Stent unterbrochen. Besonders vorteilhaft ist es, wenn nicht unterbrochene Kreuzungen 20 nicht unmittelbar benachbart sind.

Die Wandsegmente, die die Gitterstruktur bilden, weisen bei dem erfindungsgemäßen Stent für die geschilderte medizinische Indikation vorteilhafterweise von Kreuzung zu Kreuzung eine Segmentlänge n (siehe Fig. 1) von 4 mm auf. Der Winkel α (ebenfalls in Fig. 1 gekennzeichnet), unter dem die Wandsegmente an den Kreuzungen von der Längsrichtung L des Stents abweichen, beträgt vorteilhafterweise 15°, wobei die Breite c der Wandsegmente bevorzugt 0,132 mm und die Wanddicke e des Stents bevorzugt 0,2 mm beträgt.

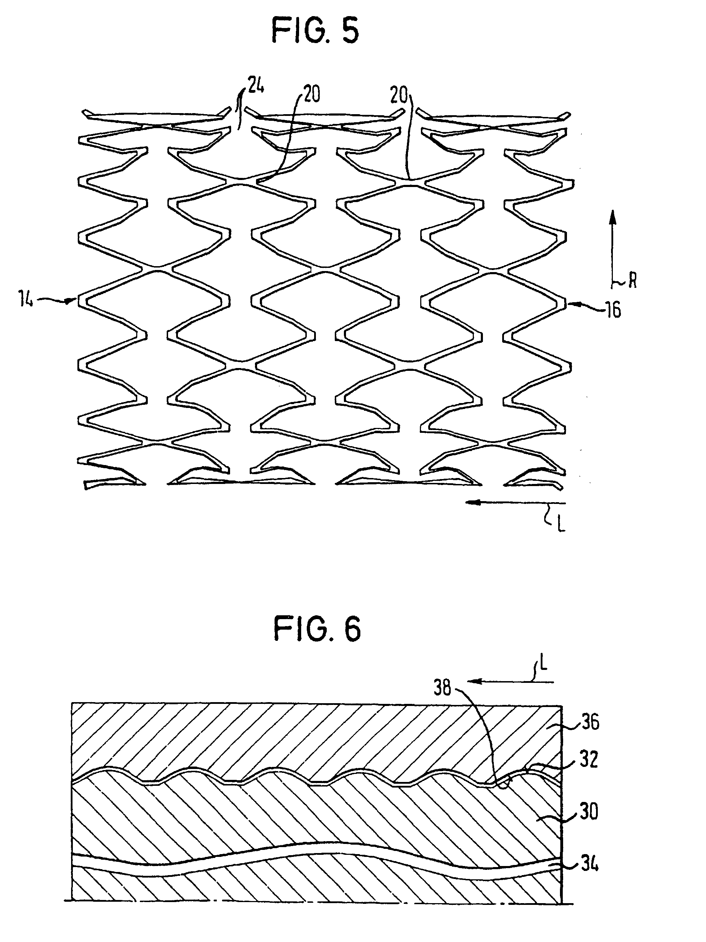

Der erfindungsgemäße Stent läßt sich in seiner Form durch ein geeignetes Formwerkzeug herstellen. Diese Herstellung wird später unter Bezugnahme auf die Fig. 6 noch ausführlicher erläutert.

Gemäß dem in den Fig. 3 und 4 dargestellten Ausführungsbeispiel sind aufgeweitete Kreuzungen 24 sowohl an sämtlichen unterbrochenen Kreuzungen 22 als auch aufgeweitete Kreuzungen 26 an den nicht unterbrochenen Kreuzungen 20 vorgesehen. Ferner ist der Fig. 3 entnehmbar, daß der erfindungsgemäße Stent asymmetrisch gestaltet ist. Diese Asymmetrie bezieht sich darauf, daß der erste Endbereich 14 des Stents in vergleichbarer Art aufgeweitet ist, wie die aufgeweiteten unterbrochenen Kreuzungen 24 und die aufgeweiteten nicht unterbrochenen Kreuzungen 26. Im Gegensatz dazu ist der zweite Endbereich 16 nicht aufgeweitet. Derart asymmetrische Konfigurationen können je nach Anwendungsbereich vorteilhaft sein. In diesem Zusammenhang sei angemerkt, daß gemäß der vorliegenden Erfindung, obwohl in den Figuren im wesentlichen zylindrische Ausführungsbeispiele dargestellt sind, auch konische, bauchige oder in Form einer Verzweigung geteilte Ausführungsbeispiele zum Einsatz kommen können.

Fig. 4 zeigt eine vergrößerte Detailansicht der Fig. 3. Der Figur läßt sich eindeutig entnehmen, wie gemäß dem bevorzugten Ausführungsbeispiel der Erfindung aufgeweitete unterbrochene Kreuzungen 24 und aufgeweitete nicht unterbrochene Kreuzungen 26 derart angeordnet sind, daß sich in Umfangsrichtung um den Stent laufende Erhebungen und Vertiefungen bilden. In Längsrichtung des Stents betrachtet ist eine sich zyklisch wiederholende Bogenform ausgebildet. Wie der Fig. entnehmbar, ist diese Bogenform derart ausgestaltet, daß die Enden der Wandsegmente an den aufgeweiteten unterbrochenen Kreuzungen im wesentlichen wieder parallel zur Längsrichtung L des Stents verlaufen. Obwohl die bogenförmige Aufweitung der Wandsegmente an den unterbrochenen und an den nicht unterbrochenen Kreuzungen bevorzugt ist, sind auch andersartig geformte Aufweitungen denkbar. Zum Beispiel ließen sich die Wandsegmente an den unterbrochenen Kreuzungen nicht bogenförmig aufweiten, sondern eher polygonartig nach außen abwinkeln, so daß die aufgeweiteten Teile der Wandsegmente geradlinig sind.

Fig. 5 gibt ein zweites Ausführungsbeispiel der vorliegenden Erfindung wieder. Gemäß diesem zweiten Ausführungsbeispiel weist der Stent ebenfalls eine Radialrichtung R und eine Längserstreckung L auf. Ein Unterschied zu dem erfindungsgemäßen Stent nach der ersten Ausführungsform besteht darin, daß der in der Fig. 5 dargestellte Stent an seinen nicht unterbrochenen Kreuzungen 20 nicht aufgeweitet ist, d.h., daß er dort gemäß seiner ursprünglichen, im wesentlichen zylindrischen Form verläuft. Die unterbrochenen Kreuzungen hingegen sind in Form von aufgeweiteten unterbrochenen Kreuzungen 24 ausgebildet. Ein weiterer Unterschied zur ersten Ausführungsform besteht darin, daß der in der Fig. 5 abgebildete Stent symmetrisch ausgebildet ist, d.h., daß er sowohl in seinem ersten Endbereich 14, als auch in seinem zweiten Endbereich 16 aufgeweitet ist. In Analogie zur Herstellung der zuerst geschilderten Ausführungsform läßt sich diese Ausführungsform ebenfalls durch ein geeignet geformtes Formwerkzeug erzeugen.

In Fig. 6 ist ein Werkzeug abgebildet, das für die Herstellung eines erfindungsgemäßen Stents geeignet ist. Im wesentlichen besteht das Werkzeug aus einem Kern 30 und aus einer Außenform 36. Der Kern 30 ist im allgemeinen im wesentlichen zylindrisch ausgebildet und weist auf seiner Außenseite eine Formfläche 32 auf. Diese Kernformfläche 32 ist, wie aus der Querschnittsansicht der Fig. 6 hervorgeht, in Richtung der Längserstreckung L des Stents annähernd sinusförmig ausgebildet. Diese Sinusform entspricht einem möglichen gewünschten Verlauf der Wandsegmente und des Stents, so daß die aufgeweiteten unterbrochenen Kreuzungen 24 und möglicherweise die aufgeweiteten nicht unterbrochenen Kreuzungen 26 bogenförmig, nämlich in Form der Sinuskurve aufgeweitet sind. Die Außenform 36 weist ebenfalls eine Formfläche 38 auf. Diese Außenformfläche 38 ist derart ausgebildet, daß sie ein Gegenstück zur Kernformfläche 32 bildet. Vorzugsweise ist die Außenform 36 teilbar ausgebildet, so daß sie in geeigneter Weise auf den Kern 30 aufgebracht werden kann.

Wie aus der Fig. 6 ebenfalls hervorgeht, kann der Kern 30 mit Kühl- oder Heizkanälen 34 versehen sein, von denen in der Figur nur einer schematisch angedeutet ist. Diese Heizkanäle können in Längsrichtung des Kerns 30 oder auch spiralförmig in Umfangsnähe des Kerns verlaufen. Es ist ebenfalls denkbar, den Kern 30, gegebenenfalls die Außenform 36 und den aufzuweitenden Stent von außen zu erhitzen und abzukühlen. Falls Kühl- bzw. Heizkanäle 34 zum Einsatz kommen, so stellt beispielsweise Öl ein geeignetes Medium dar, den Kern zu erhitzen bzw. abzukühlen. Bei dem Vorgang des Aufweitens kommt der Stent zwischen der Kernformfläche 32 und der Außenformfläche 38 zu liegen.

Im folgenden wird die Herstellung des erfindungsgemäßen Stents erläutert.

Im allgemeinen ist der Ausgangspunkt eines bevorzugten Herstellungsverfahrens für einen erfindungsgemäßen Steht ein rohrförmiges Element. Dieses rohrförmige Element weist einen Außendurchmesser auf und bestimmt ein inneres Lumen. Die Abmessungen des inneren Lumen entsprechen denen des um die Wandstärke des rohrförmigen Elements verringerten Außendurchmessers. In Längsrichtung L des Stents erstreckt er sich von einem ersten Endbereich 14 zu einem zweiten Endbereich 16. Vorzugsweise besteht das rohrförmige Element aus Nitinol®, wobei Nitinol eine Legierung ist, die aus den Legierungsbestandteilen Nickel und Titan mit den folgenden Masseanteilen besteht: 54,5 bis 57 Masseprozent Nickel und 43 bis 45,5 Masseprozent Titan. Nickel und Titangehalt sollten, mit Ausnahme einiger eventuell vorhandener Verunreinigungen, insgesam 100 Masseprozent ergeben. Nitinol® hat sich aufgrund seines stark ausgeprägten Formgedächtniseffekts, guter physikalischer und mechanischer Gebrauchseigenschaften, Korrosionsträgheit und der biologischen Verträglichkeit als für die Stomatologie und Implantologie als besonders geeignet herausgestellt.

Für radiologische Stents werden Stents ohne mehrlagige Kreuzungen der Wandsegmente bevorzugt. Ein dünnwandiger Aufbau garantiert, daß keine lumeneinschränkende Gefäßeinlage entsteht. Um die den dargestellten Stents eigene Gitterstruktur aus dem rohrförmigen Element zu erzeugen, wird dieses rohrförmige Element in einem weiteren Herstellungsschritt zu einer Gitterstruktur geschlitzt. Unter Schlitzen wird im Sinne der vorliegenden Erfindung das Erzeugen von schlitzähnlichen Öffnungen verstanden. Das Schlitzen findet in der Regel im nicht expandierten Zustand statt und die schlitzähnlichen Öffnungen werden vorzugsweise durch Laserschneiden hergestellt. Wie bereits erwähnt sind jedoch ebenfalls andere Verfahren -chemische, mechanische, elektromagnetische oder elektrische Verfahren- denkbar. Durch das Schlitzen werden schlitzähnliche Öffnungen gebildet, die von einzelnen Wandsegmenten umgeben sind. In den Bereichen, in denen zwei schlitzähnliche Öffnungen aneinander angrenzen, verzweigen sich die Wandsegmente an Kreuzungen.

Um dem Stent die gewünschte Flexibilität zu verleihen, wird vorzugsweise bei der Erzeugung der schlitzähnlichen Öffnungen zugleich eine Flexibilitätsschlitzung vorgenommen. In diesem Schritt werden einige der Kreuzungen durch Schlitzen unterbrochen. In Abhängigkeit von den gewünschten Festigkeitseigenschaften der entstehenden Struktur werden unterschiedlich viele der Kreuzungen unterbrochen. Falls gewünscht wird, daß die Flexibilität des Stents im gesamten Stentbereich homogen ist, müssen sich die unterbrochenen Kreuzungen in regelmäßiger Verteilung befinden. Wird lokal eine höhere Flexibilität gewünscht, können in den Bereichen höherer Flexibilität mehrere Kreuzungen unterbrochen werden, als in den Bereichen geringerer Flexibilität. Es hat sich als vorteilhaft herausgestellt, 2/3 aller Kreuzungen zu unterbrechen, und das Muster dieser Unterbrechung derart regelmäßig zu gestalten, daß weder in Längsrichtung, noch in Umfangsrichtung des Stents nicht unterbrochene Kreuzungen benachbart sind.

In einer besonders bevorzugten Anwendung des erfindungsgemäßen Stents ist dieser selbstexpandierend, und zwar in Abhängigkeit von seiner Umgebungstemperatur. Diese temperaturreaktive Expansion findet bei einem metallischen Stent durch die Umwandlung der kristallinen Metallgitterstruktur von einem martensitischen Gefüge in ein austenitisches Gefüge statt. Vorzugsweise ist die Bildung des austenitischen Gefüges knapp unterhalb des Körpertemperatur abgeschlossen, d.h. bei ca. 35°C. Zusätzlich zu den unmittelbar mit der Kristallgitterstruktur verbundenen Eigenschaften, wie z.B. Magnetisierbarkeit oder ähnlichem, verändert sich auch die Festigkeit des Stents vorteilhaft. Bei Erreichen des sogenannten austenitischen Plateaus weist der Stent eine höhere Festigkeit gegen Verformung auf. Diese Programmierung des Formgedächtniseffekts, so daß der Stent als Ergebnis temperaturreaktiv ist, findet in einem Wärmebehandlungsschritt statt. Bei diesem Schritt wird der Stent für eine ausreichende Zeit auf eine Temperatur erhitzt, bei der sich Versetzungen des Kristallgitters lösen. Diese Temperatur ist hier mit "Versetzungsschwellentemperatur" bezeichnet worden. Die Höhe der Temperatur hängt von den gewählten Werkstoffen ab

Vorteilhafterweise läßt sich diese Programmierung des Formgedächtniseffekts bereits mit einem Aufweiten der Wandsegmente an den gewünschten Positionen verbinden. Hierzu wird der Stent vor der Erwärmung bevorzugt auf einen Kern 30 aufgezogen, der im unteren Teil der Fig. 6 dargestellt ist. Dieser Kern weist auf seiner Außenseite eine Kernformfläche 32 auf, die in ihrer Kontur der gewünschten Endkontur des Stents entspricht. Um die Verformung des Stents zu erleichtern, ist vorteilhafterweise ebenfalls eine Außenform 36 vorgesehen. Diese Außenform 36 weist ebenfalls eine Formfläche 38 auf, wobei bei auf den Kern 30 aufgebrachter Außenform 36 der zu behandelnde Stent zwischen der Kernformfläche 32 und der Außenformfläche 38 liegt. Wie bereits erwähnt, ist die Außenform 36 bevorzugt geteilt, so daß sie, möglicherweise auch mit graduell zunehmender Kraft, auf den Kern mit Stent aufgebracht werden kann. Die aus der Zeichnung entnehmbaren, in Radialrichtung des Kerns vorgesehenen Erhöhungen auf der Kernformfläche 32 können dabei um den Kern umlaufen, der eine im wesentlichen zylindrische Form aufweist.

Im folgenden wird dann der Kern mit dem Stent und eventuell der Außenform mit geeigneten Mitteln auf die oben erwähnte Temperatur zum Programmieren des Formgedächtniseffekts gebracht.

Nach entsprechender Abkühlung kann der Stent von Außenform und Kern entfernt werden, indem die Außenform entfernt und der Stent von dem Kern abgezogen wird. Die Elastizität und Flexibilität des Stents erleichtert das Entfernen desselben von dem Kern 30, ohne Kern und vor allem Stent zu beschädigen.

Um eventuelle Oberflächenunregelmäßigkeiten, wie bereits vorhandene oder im Herstellungsverfahren entstandene Kratzer, Grate oder ähnliches zu beseitigen, ist vorgesehen, daß der Stent poliert wird, bevor er in ein der medizinischen Indikation entsprechendes Applikationssystem eingebaut, vorzugsweise eingezogen wird. Ein solches Applikationssystem kann z.B. eines sein, mit dem sich eine perkutane transluminale Angioplastie vornehmen läßt und dient als Beispiel für ein System gemäß der Seldinger Methode zur retrograden beziehungsweise antegraden Katheterisierung von Körpergefäßen.

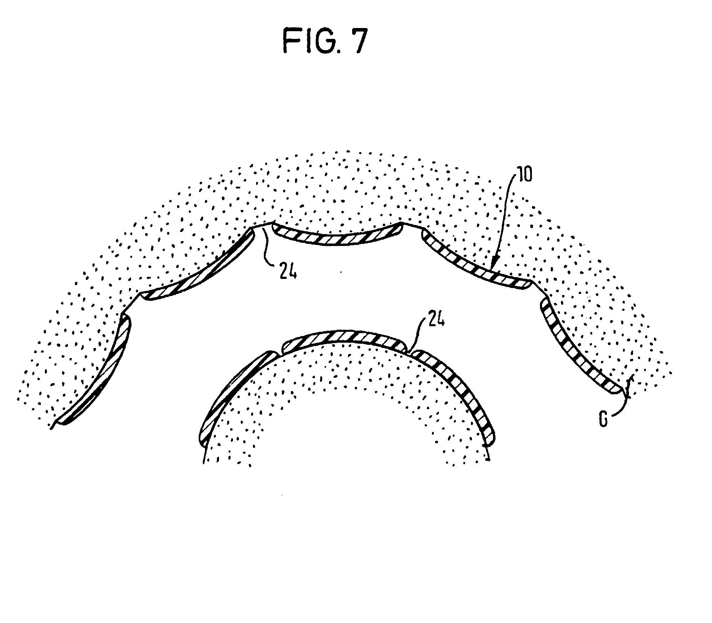

Im folgenden wird unter Bezugnahme auf die Fig. 7 die Wirkung des erfindungsgemäßen Stents geschildert.

In der Fig. 7 ist schematisch dargestellt, wie der erfindungsgemäße Stent 10 sich in einem gekrümmten Gefäß G verhält. Abgebildet ist der Stent in einer Phase, in der er sich nach dem Expansionsvorgang an die Wandung eines Gefäßes G angelegt hat oder an sie angelegt wurde. Es wird deutlich, wie sich die aufgeweiteten unterbrochenen Kreuzungen 24 auf der Innenseite der Krümmung in ihrem Verlauf und ihrer Form gleichmäßig an die Gefäßwandung anpassen. Auch auf der krümmungsäußeren Seite ragen die Wandsegmente an den unterbrochenen Kreuzungen 24 nicht in das innere Lumen des Katheters, sondern sind zum umgebenden Gefäßgewebe hin gerichtet. Sie ragen ausreichend weit in das Gefäßgewebe, so daß die Wandsegmente zwischen den Kreuzungen sanft an der Gefäßwandung anliegen.

Die Aufweitung des Stents hat die Wirkung, daß das innere Lumen des Gefäßes auch in Bereichen starker Krümmung, insbesondere an der krümmungsinneren Seite, nicht dadurch verkleinert wird, daß Wandsegmente in das Lumen ragen. Zudem ist eine Lageveränderung des Stent erschwert, da sich die in Richtung des Gefäßgewebes zeigenden Wandsegmente, insbesondere an der krümmungsäußeren Seite, in dem Gefäßgewebe verankern.

## Patentansprüche

1. Expandierbarer Stent (10), umfassend
- eine elastische rohrförmige Gitterstruktur mit einem ersten Endbereich (14), einem zweiten Endbereich (16), einer Längsrichtung (L) und einer Radialrichtung (R), wobei
- die Gitterstruktur einen Außendurchmesser und ein inneres Lumen bestimmt und von Wandsegmenten gebildet ist, die sich an Kreuzungen (20) verzweigen, und
- die Gitterstruktur an zumindest einigen der Kreuzungen (22) unterbrochen ist, um die Flexibilität des Stents zu erhöhen,
**dadurch gekennzeichnet, daß**
- die Wandsegmente (24) zumindest an den unterbrochenen Kreuzungen in der Radialrichtung derart aufgeweitet sind, daß bei Krümmung des Stents entlang der Längsrichtung eine Verkleinerung des inneren Lumens durch die Wandsegmente an den unterbrochenen Kreuzungen verhindert ist.

2. Stent nach Anspruch 1, **dadurch gekennzeichnet, daß** weiterhin die Wandsegmente in dem ersten (14) und/oder dem zweiten Endbereich (16) in der Radialrichtung (R) aufgeweitet sind.

3. Stent nach mindestens einem der vorhergehenden Ansprüche, **dadurch gekennzeichnet, daß** die Aufweitung der Wandsegmente durch eine bogenförmige Krümmung dieser Wandsegmente entlang der Längsrichtung gebildet ist.

4. Stent nach mindestens einem der vorhergehenden Ansprüche, **dadurch gekennzeichnet, daß** die Wandsegmente in einer regelmäßigen Verteilung über den Stent an im wesentlichen zwei Drittel aller Kreuzungen unterbrochen sind.

5. Stent nach mindestens einem der vorhergehenden Ansprüche, **dadurch gekennzeichnet, daß** die Gitterstruktur im expandierten Zustand des Stents Öffnungen mit einer Öffnungsweite von maximal 9 mm aufweist.

6. Stent nach mindestens einem der vorhergehenden Ansprüche, **dadurch gekennzeichnet, daß** die Wandsegmente eine Breite zwischen 0,12 mm und 0,17 mm aufweisen.

7. Stent nach mindestens einem der vorhergehenden Ansprüche, **dadurch gekennzeichnet, daß** die Gitterstruktur im wesentlichen eine Wandstärke von zwischen 0,2 mm und 0,3 mm aufweist.

8. Stent nach mindestens einem der vorhergehenden Ansprüche, **dadurch gekennzeichnet, daß** der Stent aus einem metallischen Werkstoff besteht.

9. Stent nach Anspruch 8, **dadurch gekennzeichnet, daß** der Stent aus einem metallischen Werkstoff mit Formgedächtnis besteht.

10. Stent nach Anspruch 9, **dadurch gekennzeichnet, daß** der metallische Werkstoff aus einer Legierung besteht, die Nickel und Titan enthält.

11. Stent nach Anspruch 10, **dadurch gekennzeichnet, daß** in der Legierung des Stents folgende Legierungsanteile enthalten sind:
- Nickel: 54,5 bis 57 Masseprozent,
- Titan: 43 bis 45,5 Masseprozent.

12. Herstellungsverfahren für einen Stent mit folgenden Schritten:
- Bereitstellen eines rohrförmigen Elements mit einem Außendurchmesser, einem inneren Lumen, einem ersten Endbereich und einem zweiten Endbereich;
- Schlitzen des rohrförmigen Elements zu einer Gitterstruktur, wobei die Gitterstruktur von Wandsegmenten gebildet ist, die sich an Kreuzungen verzweigen;
- Unterbrechen von zumindest einigen Kreuzungen an ausgewählten Positionen, um die Flexibilität des Stents zu erhöhen;
- Aufweiten der Wandsegmente zumindest an den unterbrochenen Kreuzungen in der Radialrichtung derart, daß bei Krümmung des Stents entlang der Längsrichtung eine Verkleinerung des inneren Lumens durch die Wandsegmente an den unterbrochenen Kreuzungen verhindert ist.

13. Verfahren nach Anspruch 12, **dadurch gekennzeichnet, daß** der Schritt des Aufweitens das Aufweiten der Wandsegmente in der Radialrichtung an dem ersten und dem zweiten Endbereich umfaßt.

14. Verfahren nach Anspruch 12 oder 13, **dadurch gekennzeichnet, daß** die Verfahrensschritte in der angeführten Reihenfolge durchgeführt werden.

15. Verfahren nach mindestens einem der Ansprüche 12 bis 14, wobei der Stent aus einem metallischem Werkstoff besteht und das Verfahren **dadurch gekennzeichnet ist, daß** der Stent bei dem oder nach dem Schritt des Aufweitens der Wandsegmente wärmebehandelt wird, um einen temperaturreaktiven Formgedächtniseffekt im Bereich der aufgeweiteten Wandsegmente zu erzielen.

16. Verfahren nach Anspruch 15, **dadurch gekennzeichnet, daß** das Verfahren zwischen den Schritten des Schlitzens des rohrförmigen Elements und des Unterbrechens der Kreuzungen weiterhin einen Schritt des Beeinflussens der Struktur des Metallgitters des Stents umfaßt.

17. Verfahren nach mindestens einem der Ansprüche 12 bis 16, **dadurch gekennzeichnet, daß** das Verfahren vor dem Schritt des Unterbrechens der Kreuzungen zusätzlich einen Schritt des Wärmebehandelns umfaßt, um einen temperaturreaktiven Formgedächtniseffekt im gesamten Stentbereich zu erzielen.

18. Verfahren nach mindestens einem der Ansprüche 12 bis 17, **dadurch gekennzeichnet, daß** das Verfahren zuletzt weiterhin einen Schritt des Polierens des Stents umfaßt.

19. Verfahren nach mindestens einem der Ansprüche 12 bis 18, **dadurch gekennzeichnet, daß** das Unterbrechen der Kreuzungen in dem Schritt des Schlitzen erfolgt.

20. Verfahren nach mindestens einem der Ansprüche 12 bis 19, **dadurch gekennzeichnet, daß** die Schritte des Schlitzens durch Laserschneiden vorgenommen werden.

21. Verfahren nach mindestens einem der Ansprüche 12 bis 20, **dadurch gekennzeichnet, daß** der Schritt des Aufweitens folgende Teilschritte umfaßt:
- Aufbringen des Stents auf einen Dorn, wobei der Dorn als Gegenform zur aufgeweiteten Form des Stents ausgebildet ist;
- Erhitzen des auf den Dorn gebrachten Stents;
- Abkühlen des erhitzten Stents;
- Entfernen des abgekühlten Stents von dem Dorn.

22. Verfahren nach Anspruch 21, **dadurch gekennzeichnet, daß** vor dem Schritt des Entfernens des abgekühlten Stents von dem Dorn ein Formelement außen über den Dorn und den Stent gebracht wird, das in seiner Kontur der aufgeweiteten Form des Stents entspricht.

23. Verfahren nach einem der Ansprüche 21 oder 22, wobei der Stent aus einem metallischen Werkstoff mit einer Versetzungsschwellentemperatur besteht und der Stent in dem Teilschritt des Erhitzens auf eine Temperatur oberhalb der Versetzungsschwellentemperatur erhitzt und in dem Schritt des Abkühlens auf eine Temperatur unterhalb der Versetzungsschwellentemperatur abgekühlt wird.

24. Stent nach mindestens einem der Ansprüche 1 bis 11 in Kombination mit einem Applikations-System.

25. Stent nach Anspruch 24, **dadurch gekennzeichnet, daß** das Applikations-System einen Ballon-Dilatationskatheter enthält.

26. Stent nach einem der Ansprüche 24 oder 25, **dadurch gekennzeichnet, daß** das Applikations-System ein System gemäß der Seldinger Methode zur Katheterisierung von Körpergefäßen ist.

## Claims

1. Expandable stent (10) comprising
- an elastic tubular lattice structure having a first end region (14), a second end region (16), a longitudinal direction (L) and a radial direction (R), wherein
- the lattice structure determines an outer diameter and an internal passageway and is formed by wall segments which branch at intersections (20) and
- the lattice structure is broken at least at some of the intersections (22) in order to increases the flexibility of the stent,
**characterised in that**
- the wall segments (24) at least at the broken intersections are widened in the radial direction in such a way that on bending the stent along the longitudinal direction a diminution of the internal passageway is prevented by the wall segments at the interrupted intersections.

2. Stent according to Claim 1, **characterised in that**, furthermore, the wall segments in the first (14) and/or the second end region (16) are widened in the radial direction (R).

3. Stent according to at least one of the preceding claims, **characterised in that** the widening of the wall segments is formed by an arc-shaped curvature of these wall segments along the longitudinal direction.

4. Stent according to at least one of the preceding claims, **characterised in that** breaks in the wall segments are regularly distributed over the stent at substantially two thirds of all intersections.

5. Stent according to at least one of the preceding claims, **characterised in that** the lattice structure in the expanded state of the stent possesses openings having an aperture width of at most 9 mm.

6. Stent according to at least one of the preceding claims, **characterised in that** the wall segments have a width of between 0.12 mm and 0.17 mm.

7. Stent according to at least one of the preceding claims, **characterised in that** the lattice structure substantially has a wall thickness of between 0.2 mm and 0.3 mm.

8. Stent according to at least one of the preceding claims, **characterised in that** the stent consists of a metallic material.

9. Stent according to Claim 8, **characterised in that** the stent consists of a metallic material having shape memory.

10. Stent according to Claim 9, **characterised in that** the metallic material consists of an alloy containing nickel and titanium.

11. Stent according to Claim 10, **characterised in that** in the alloy of the stent the following alloy proportions are present:
- nickel: 54.5 to 57 per cent by weight
- titanium: 43 to 45.5 per cent by weight.

12. Production method for a stent having the following steps:
- preparation of a tubular element having an outer diameter, an internal opening, a first end region and a second end region;
- slitting the tubular element to form a lattice structure, wherein the lattice structure is formed by wall segments which branch at intersections;
- breaking at least some intersections at selected positions in order to increase the flexibility of the stent;
- widening the wall segments at least at the broken intersections in the radial direction in such a way that on bending the stent along the longitudinal direction a diminution of the internal opening is prevented by the wall segments at the broken intersections.

13. Method according to Claim 12, **characterised in that** the widening step comprises the widening of the wall segments in the radial direction at the first and the second end region.

14. Method according to Claim 12 or 13, **characterised in that** the steps in the method are carried out in the stated sequence.

15. Method according to at least one of Claims 12 to 14, wherein the stent consists of a metallic material and the method is **characterised in that** in or after the step for widening the wall segments the stent is heat-treated in order to achieve a temperature-reactive shape-memory effect in the region of the widened wall segments.

16. Method according to Claim 15, **characterised in that** between the steps of slitting the tubular element and breaking the intersections the method, furthermore, comprises a step for influencing the structure of the metal lattice of the stent.

17. Method according to at least one of Claims 12 to 16, **characterised in that** prior to the step of breaking the intersections the method additionally comprises a heat-treatment step in order to achieve a temperature-reactive shape-memory effect over the whole area of the stent.

18. Method according to at least one of Claims 12 to 17, **characterised in that** the method further finally comprises a step for polishing the stent.

19. Method according to at least one of Claims 12 to 18, **characterised in that** the breaking of the intersections ensues in the slitting step.

20. Method according to at least one of Claims 12 to 19, **characterised in that** the slitting steps are done by laser cutting.

21. Method according to at least one of Claims 12 to 20, **characterised in that** the widening step comprises the following substeps:
- attaching the stent to a mandrel, wherein the mandrel is constructed as a countermould to the widened shape of the stent;
- heating the stent fitted on the mandrel;
- cooling the heated stent; and
- removal of the cooled stent from the mandrel.

22. Method according to Claim 21, **characterised in that** prior to the step of removing the cooled stent from the mandrel a moulding element is fitted outside over the mandrel and the stent which in contour matches the widened shape of the stent.

23. Method according to one of Claims 21 or 22, wherein the stent consists of a metallic material having a dislocation threshold temperature and the stent is heated in the heating substep to a temperature above the dislocation threshold temperature and cooled in the cooling step to a temperature below the dislocation threshold temperature.

24. Stent according to at least one of Claims 1 to 11 in combination with an application system.

25. Stent according to Claim 24, **characterised in that** the application system contains a balloon dilatation catheter.

26. Stent according to one of Claims 24 or 25, **characterised in that** the application system is a system according to the Seldinger method for the catheterisation of vessels in the body.

## Revendications

1. Stent (10) expansible comprenant
- une structure à grille tubulaire élastique avec une première zone d'extrémité (14), une deuxième zone d'extrémité (16), une direction longitudinale (L) et une direction radiale (R),
- la structure à grille définissant un diamètre extérieur et une lumière intérieure et étant formée par des segments de parois qui se ramifient au niveau de croisements (20), et
- la structure à grille étant sectionnée au niveau d'au moins quelques-uns des croisements (20) en vue d'augmenter la flexibilité du stent,
**caractérisé en ce que**
- les segments de paroi (24), au moins au niveau des croisements sectionnés, sont élargis dans la direction radiale de telle sorte que, en cas de pliage du stent le long de la direction longitudinale, un rétrécissement de la lumière intérieure par les segments de paroi est empêché au niveau des croisements sectionnés.

2. Stent selon la revendication 1, **caractérisé en ce que**, par ailleurs, les segments de paroi dans la première zone d'extrémité (14) et/ou la deuxième zone d'extrémité (16) sont élargis dans la direction radiale (R).

3. Stent selon au moins l'une des revendications précédentes, **caractérisé en ce que** l'élargissement des segments de paroi est formé par un pliage en courbe de ces segments de paroi le long de la direction longitudinale (L).

4. Stent selon au moins l'une des revendications précédentes, **caractérisé en ce que** les segments de paroi sont sectionnés de manière régulièrement répartie sur le stent au niveau d'à peu près deux tiers de la totalité des croisements.

5. Stent selon au moins l'une des revendications précédentes, **caractérisé en ce que**, à l'état dilaté du stent, la structure à grille comporte des ouvertures avec une largeur d'ouverture de 9 mm maximum.

6. Stent selon au moins l'une des revendications précédentes, **caractérisé en ce que** les segments de paroi ont une largeur comprise entre 0,12 mm et 0,17 mm.

7. Stent selon au moins l'une des revendications précédentes, **caractérisé en ce que** la structure à grille possède sensiblement une épaisseur de paroi comprise entre 0,2 mm et 0,3 mm.

8. Stent selon au moins l'une des revendications précédentes, **caractérisé en ce que** le stent est réalisé dans un matériau métallique.

9. Stent selon la revendication 8, **caractérisé en ce que** le stent est réalisé dans un matériau métallique à mémoire de forme.

10. Stent selon la revendication 9, **caractérisé en ce que** le matériau métallique est formé par un alliage contenant du nickel et du titane.

11. Stent selon la revendication 10, **caractérisé en ce que** l'alliage du stent contient les éléments d'alliage suivants :
- nickel : 54,5 à 57 % en poids,
- titane : 43 à 45,5 % en poids.

12. Procédé de fabrication d'un stent, comprenant les étapes suivantes :
- préparation d'un élément tubulaire avec un diamètre extérieur, une lumière intérieure, une première zone d'extrémité et une deuxième zone d'extrémité ;
- entaillage de l'élément tubulaire pour obtenir une structure à grille, la structure à grille étant formée par des segments de paroi, qui se ramifient au niveau de croisements ;
- sectionnement d'au moins quelques croisements à des emplacements sélectionnés, afin d'augmenter la flexibilité du stent ;
- élargissement des segments de paroi dans la direction radiale au moins au niveau des croisements sectionnés, de telle sorte que, en cas de pliage du stent le long de la direction longitudinale, un rétrécissement de la lumière intérieure par les segments de paroi est empêché au niveau des croisements sectionnés.

13. Procédé selon la revendication 12, **caractérisé en ce que** l'étape d'élargissement englobe l'élargissement des segments de paroi dans la direction radiale au niveau de la première zone d'extrémité et de la deuxième zone d'extrémité.

14. Procédé selon la revendication 12 ou 13, **caractérisé en ce que** les étapes du procédé sont mises en oeuvre dans l'ordre présenté.

15. Procédé selon au moins l'une des revendications 12 à 14, dans lequel le stent est réalisé dans un matériau métallique et le procédé est **caractérisé en ce que** le stent, pendant ou après l'étape d'élargissement des segments de paroi, est soumis à un traitement thermique en vue d'obtenir, dans la zone des segments de paroi élargis, un effet de mémoire de forme réagissant à la température.

16. Procédé selon la revendication 15, **caractérisé en ce que** le procédé, entre les étapes d'entaillage de l'élément tubulaire et de sectionnement des croisements, comporte en outre une étape destinée à influer sur la structure de la grille métallique du stent.

17. Procédé selon au moins l'une des revendications 12 à 16, **caractérisé en ce que** le procédé, avant l'étape de sectionnement des croisements, comprend en outre une étape de traitement thermique destinée à obtenir dans toute la zone du stent un effet de mémoire de forme réagissant à la température.

18. Procédé selon au moins l'une des revendications 12 à 17, **caractérisé en ce que** le procédé comprend enfin également une étape de polissage du stent.

19. Procédé selon au moins l'une des revendications 12 à 18, **caractérisé en ce que** le sectionnement des croisements est effectué pendant l'étape d'entaillage.

20. Procédé selon au moins l'une des revendications 12 à 19, **caractérisé en ce que** les étapes d'entaillage sont exécutées par découpe au laser.

21. Procédé selon l'une quelconque des revendications 12 à 20, **caractérisé en ce que** l'étape d'élargissement comporte les étapes partielles suivantes :
- pose du stent sur un mandrin, le mandrin étant conçu comme une forme complémentaire de la forme élargie du stent ;
- chauffage du stent posé sur le mandrin ;
- refroidissement du stent chauffé ;
- enlèvement du stent refroidi du mandrin.

22. Procédé selon la revendication 21, **caractérisé en ce que**, avant l'étape d'enlèvement du stent refroidi du mandrin, on pose de l'extérieur, au-dessus du mandrin et du stent, un élément moulé, dont le contour correspond à la forme élargie du stent.

23. Procédé selon la revendication 21 ou 22, dans lequel le stent est réalisé dans un matériau métallique avec un seuil de température de dislocation et le stent, dans l'étape partielle du chauffage, est chauffé à une température supérieure au seuil de température de dislocation et, dans l'étape du refroidissement, est refroidi à une température inférieure au seuil de température de dislocation.

24. Stent selon au moins l'une des revendications 1 à 11 en combinaison avec un système d'implantation.

25. Stent selon la revendication 24, **caractérisé en ce que** le système d'implantation comprend un cathéter de dilatation monté sur ballonnet.

26. Stent selon la revendication 24 ou 25, **caractérisé en ce que** le système d'implantation est un système selon la technique de Seldinger, destinée à la mise en place de cathéters dans des vaisseaux du corps humain.
